# EUROPEAN PATENT APPLICATION

(11) **EP 4 420 620 A1**
(43) Date of publication of application: **28.08.2024**
(21) Application number: 22883031.1
(22) Date of filing: 21.10.2022
(51) Int. Cl.: A61B 17/17

(54) **OSTEOTOMY GUIDE**

(30) Priority: 22.10.2021 ES 202132077 U
(71) Applicant: Servicio Andaluz de Salud, 41071 Sevilla (ES)
(72) Inventor: GARCIA PRIEGO, Alfonso Luis, 41071 Sevilla (ES); QUESADA GONZALEZ, Antonio, 41071 Sevilla (ES); SÁNCHEZ HUELTES, Victor Manuel, 41071 Sevilla (ES); BONILLA ALARCÓN, José Ramón, 41071 Sevilla (ES)
(74) Representative: Hoffmann Eitle
(86) International application number: PCT/ES2022/070684
(87) International publication number: WO 2023/067231

(57) **Abstract**

The present invention relates to a osteotomy guide (1), characterised in that it comprises an elongated body (2) having an upper surface (2a) and a lower surface (2b), and a slot (3) longitudinally arranged in the elongated body (2); wherein the slot (3) is for passage between the upper and lower surfaces (2a, 2b), to allow the passage of a chop saw through the slot and to guide the travel thereof; and wherein the slot (3) has a plurality of narrowings (5) therein that have the width of the slot.

## Description

### Object of the Invention

The present invention relates to a guide for performing osteotomies, i.e., for making cuts on bones, preferably applicable for making cuts on the first metatarsal in hallux valgus (bunion) surgeries.

An object of the invention is to enable performing different types of osteotomies such as, for example: scarf, short scarf, Chevron osteotomy, etc., with a single guide.

An additional object of the invention is to provide a guide for osteotomy which allows modifying the cutting angle in a simple manner as desired by the surgeon who is performing the intervention.

### Background of the Invention

A guide system for bunion surgeries from the manufacturer, Wishbone Medical, is known, said system consisting of a set of guides, each of them particularly designed for a specific type of osteotomy, so the surgeon has to choose the suitable type of guide for each intervention, complicating the performance of interventions of this type.

Moreover, guides of this type, which are already known, present other drawbacks since they are large in size, have a complex design, and a fixed cutting angle, greatly limiting the types of cuts that can be made.

### Description of the Invention

The invention solves the problems set forth above by providing an osteotomy guide which can be used in hallux valgus (bunion) surgery of the foot, as an instrument to make different types of bone cuts in a precise manner, without having to change the guide for each type of cut.

More specifically, the invention relates to an osteotomy guide comprising: an elongated body having an upper surface and a lower surface, and a slot longitudinally arranged in the elongated body, such that the slot is for passage between the upper and lower surfaces, to allow the passage of a chop saw through the slot and to guide the travel thereof.

The guide has stops which delimit the travel of a saw within the slot, so as to thereby adapt the guide to the desired cutting length in each surgical intervention.

Preferably, these stops consist of a plurality of narrowings of the width of the slot distributed along same and a pair of needles which can be inserted transversely into the narrowings of the slot and intended to be driven into the bone of the patient. The diameter of the needles and the dimension of the narrowings are such that the needles are retained by pressure, so that the movement of the guide is prevented, and such that the needles delimit the travel of a chop saw.

Preferably, the narrowings are materialised by means of a plurality of corrugations formed in both inner walls of the slot.

There are two types of corrugations, short corrugations and long corrugations, such that the short and long corrugations are intercalated.

The guide has at least one handle attached to the elongated body to hold the guide during the cutting process, thereby providing greater stability thereto and preventing the oscillating movement of the guide upon passage of the chop saw.

In a preferred embodiment, the handle consists of respective rods projecting respectively from each of the ends of the elongated body.

At least one of the upper or lower surfaces is flat in order to be supported on the bone material. Preferably, the upper or lower surfaces are flat and parallel to one another.

Therefore, some of the main advantages provided by the osteotomy guide of the present invention are as follows:
- unlike the known guides, the guide of the invention allows performing different osteotomies (scarf, short scarf, Chevron osteotomy) with a single guide;
- it allows modifying the cutting angle according to the surgeon's preference by simply rotating the guide;
- it is of a smaller size than the known guides;
- it incorporates two handles that provide stability, for preventing the guide from moving while performing bone cutting (osteotomy).

### Description of the Figures

A preferred embodiment of the invention is described below in reference to the figures of the application, in which the following is shown:

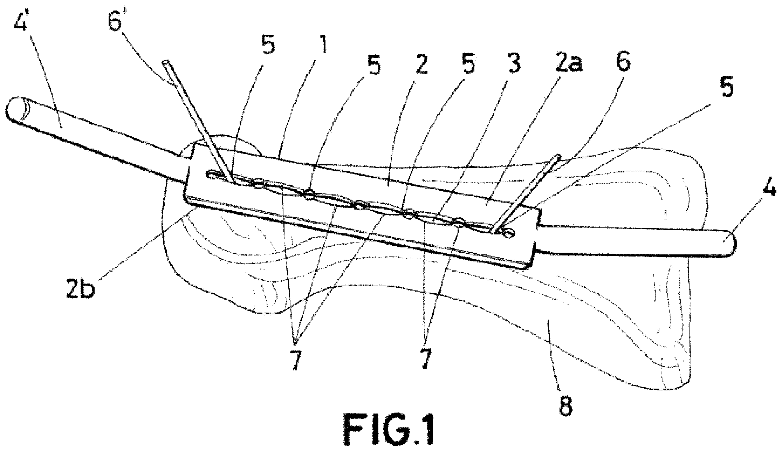

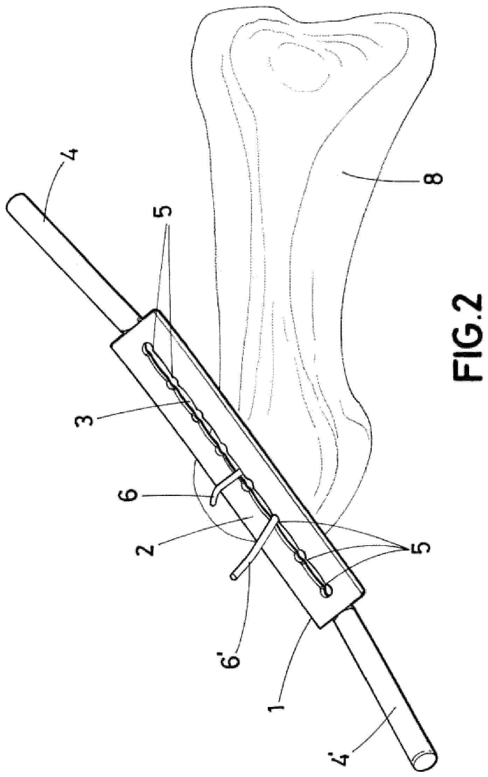

Figures 1 and 2 show respective perspective views of the osteotomy guide object of the invention, in two different usage positions for making a cut on the bone of a patient, at different cutting angles and with different cutting lengths.

### Preferred Embodiment of the Invention

In view of Figures 1 and 2, it can be seen how in this preferred embodiment the osteotomy guide (1) comprises an elongated body (2) having an upper surface (2a) and a lower surface (2b), and a slot (3) longitudinally arranged in the elongated body (2), such that the slot (3) is for passage between the upper and lower surfaces (2a, 2b) to allow the passage of a chop saw (not depicted) through the slot (3) and to guide the travel thereof.

In this embodiment, the elongated body (2) is generically flat and has a rectangular prism shape, such that the upper or lower surfaces (2a, 2b) are flat and parallel to one another. The slot (3) is arranged longitudinally in the centre of the elongated body (2) and extends over most of the length thereof.

The osteotomy guide (1) has two rods (4, 4') projecting respectively, and in an inclined manner, from each of the ends of the elongated body (2). These rods (4, 4') serve as a handle to hold the guide (1) during the cutting process.

The width of the slot (3) is variable, such that the slot has a plurality of narrowings (5) distributed along same.

The guide (1) is complemented with a pair of needles (6, 6') intended for being inserted transversely in the slot (3) so as to be driven into the bone material (8) of a patient. The needles (6, 6') can be snugly inserted in the narrowings (5) of the slot (3), such that they are retained by pressure. Therefore, the needles (6, 6') serve to fix the guide (1) on the bone material (8) in the desired position, and as stops to delimit the travel of a chop saw.

As depicted in Figures 1 and 2, the same guide (1) serves for making many types of cuts, in relation to the position, orientation or angle, and length thereof. The surgeon only needs to drive the needles (6, 6') in to delimit the cut to be made and place the guide (1) by passing the needles (6, 6') through the slot, causing the needles to coincide with the narrowings (5) so that the guide is retained.

These narrowings (5) that have the width of the slot are formed by a plurality of corrugations (7) formed in the two inner walls of the slot (3).

The guide (1) can be manufactured with a surgical material suitable for this use.

Preferably, there are short corrugations and long corrugations which are intercalated with respect to one another.

## Claims

1. An osteotomy guide (1), **characterised in that** it comprises:
an elongated body (2) having an upper surface (2a) and a lower surface (2b),
a slot (3) longitudinally arranged in the elongated body (2), and wherein the slot (3) is for passage between the upper and lower surfaces (2a, 2b), to allow the passage of a chop saw through the slot and to guide the travel thereof,
and wherein the slot (3) has a plurality of narrowings (5) therein that have the width of the slot.

2. The osteotomy guide according to claim 1, **characterised in that** it has at least one handle attached to the elongated body (2) to hold the guide (1) during the cutting process.

3. The osteotomy guide according to claim 2, **characterised in that** the elongated body (2) has two ends, and **in that** the handle consists of respective rods (4, 4') projecting respectively from each of the ends.

4. The osteotomy guide according to any of the preceding claims, **characterised in that** at least the upper or lower surfaces are flat.

5. The osteotomy guide according to any of the preceding claims, **characterised in that** the upper or lower surfaces are flat and parallel to one another.

6. The osteotomy guide according to any of the preceding claims, **characterised in that** the elongated body is generically flat and has a rectangular prism shape, and **in that** the slot is arranged longitudinally in the centre thereof.

7. The osteotomy guide according to any of claims 3 and 6, **characterised in that** the rods are inclined with respect to the elongated body.

8. The osteotomy guide according to any of the preceding claims, **characterised in that** the inner walls of the slot have a plurality of corrugations (7) forming said narrowings (5).

9. The osteotomy guide according to claim 8, **characterised in that** the inner walls of the slots have short corrugations and long corrugations, such that the short and long corrugations are intercalated.

10. The osteotomy guide according to any of the preceding claims, **characterised in that** it further comprises a pair of needles (6,6') which can be inserted transversely into the narrowings (5) of the slot (3) in order to delimit the travel of a chop saw.
